# EUROPEAN PATENT SPECIFICATION

(11) **EP 1 174 030 B1**
(45) Date of publication and mention of the grant of the patent: **21.01.2004**
(21) Application number: 00910887.9
(22) Date of filing: 23.03.2000
(51) Int. Cl.: A01N 63/02, C12N 1/20

(54) **NOVEL PANTOEA AGGLOMERANS (ERWINIA HERBICOLA) BACTERIA STRAIN AND ITS UTILIZATION AS BIOLOGICAL CONTROL AGENT OF FUNGAL DISEASES IN FRUITS**
NEUER PANTOEA AGGLOMERANS (ERWINIA HERBICOLA) BAKTERIENSTAMM UND SEINE VERWENDUNG ALS BIOLOGISCHES BEKÄMPFUNGSMITTEL VON PILZLICHEN ERKRANKUNGEN BEI FRÜCHTEN
NOUVELLE SOUCHE (PANTOEA AGGLOMERANS, (ERWINIA HERBICOLA)) ET SON UTILISATION COMME AGENT DE CONTROLE BIOLOGIQUE DES MYCOSES DES FRUITS

(30) Priority: 26.03.1999 ES 9900612
(43) Date of publication of application: 23.01.2002
(73) Proprietor: INSTITUT DE RECERCA I TECNOLOGIA AGROALIMENTARIES (IRTA), 08008 Barcelona (ES)
(72) Inventor: Vinas Almenar, Inmaculada, 25198 Lleida (ES); Usall I Rodie, Josep, 25198 Lleida (ES); Alves Afonso Nunes, Carla Alexandra, 25198 Lleida (ES); Teixido I Espasa, Neus, 25198 Lleida (ES)
(74) Representative: SUGRANES - VERDONCES - FERREGÜELA
(86) International application number: PCT/ES2000/000101
(87) International publication number: WO 2000/057706

(56) References cited:
- US-A- 5 783 411
- BRYK H. ET AL.: 'Antagonistic effect of erwinia herbicola on in vitro spore germination and germ tube elongation of botrytis cinerea and penicillium expansum' BIOCONTROL vol. 43, 1998, pages 97 - 106, XP000995992
- SHOLBERG P.L. ET AL.: 'Biological control of postharvest pathogens of apple with antagonistic microorganisms' PHYTOPATHOLOGY vol. 84, no. 10, 1994, page 1152, XP000986240
- GULATI M.K. ET AL.: 'Biocontrol of phytophtora-diseases on strawberry with bacterial antagonist' MITT. BIOL. BUNDESANSTALT LANDFORSTWIRTSCHAFT vol. 357, 1998, page 338, XP000995993
- DOCK L.L. ET AL.: 'Biological control of botrytis cinerea growth on apples stored under modified atmospheres' JOURNAL OF FOOD PROTECTION vol. 61, no. 12, 1998, pages 1661 - 1665, XP000995983
- EL-GOORANI ET AL.: 'Antibacterial and antifungal spectra of antibiotics. Produced by different strains of erwinia herbicola (=Pantoca agglomerans)' J. PHYTOPATHOLOGY vol. 136, 1992, pages 335 - 339, XP000986260

## Description

### Field of the invention

The present invention refers to a new strain of the bacterium *Pantoea agglomerans* and its use as an antagonist for the biological control of fungic diseases on fruits with the object of preventing their rotting.

### State of the art

Post-harvest diseases of fruits cause annual losses in agriculture estimated at a quantity of the order of 15% to 20% of total harvests worldwide.

The most widely used system at present for combatting the fungi which cause the rotting of the fruits is chemical control by means of treatment of the harvests with fungicidal products. The use of fungicides is quite widespread throughout the world, being estimated to represent up to 26% of the pesticides in the European and Asiatic markets, and up to 6% in the North American market.

The large-scale utilization of said fungicides products has led to a number of problems, such as the emergence of resistant pathogenic strains - which leads to a steady rise in the cost of treatments and to increased losses due to rotting - and an increase of fungicide residues on the fruits, which gives rise to health problems and hinders exports to countries which have restrictive health regulations in this sphere.

Faced with this problem and the growing demand by consumers of products free from residues, research work began a relatively few years ago in order to find new alternative methods for the control of fruit diseases, with one of the most notable development paths lying in biological control of the fungi which cause the rotting by the use of microorganisms. Said biological control is based on the inhibiting action of some microorganisms on the growth and action of the pathogenic fungi. Thus, Janisiewicz W.J. and Marchi, A., Plant Disease **76**:555 (1992), reported the use of *Pseudomonas syringae* as a post-harvest biological control for combatting pear rotting; Huang, Y.; Wild, B.L. and Morris, S.C., Annals Applied Biological **120**:367-372 (1992), reported the use of *Bacillus pumilus* as a post-harvest biological agent to combat green mould *(Penicillium digitatum)* in citrus fruits.

The use of different yeast strains as biological control agents for diseases in post-harvest fruits has been described. Thus, Viñas, I.; Usall, J.; Teixidó, N. and Sanchis, V., International Journal of Food Microbiology 40:9-16 (1998), have described the use of *Candida sake* as a biological control agent of the main post-harvest pathogenic fungi of apples and of patent applications WO-92/18009 and WO-96/30495, the latest equivalent to the Spanish patent ES-2089981, and of the United States patent US-7-745796, published as report NTIS (National Technical Information Service) number PB92-102334, also described the use of strains of *Candida* type with the aforementioned objective.

However, the bacteria agents are of particular interest as biological control agents as they are of an easy and quick growing and application and because a set of formulations for bacteria which permits their survival once applied is well-known by experts.

Bryck H. et al. *Biocontrol,* 1998, Vol. 43, pages 97-106, describe that two *Erwinia herbicola* strains isolated in Poland from leaves of apple trees, called B66 and B90, showed certain "in vitro" activity against *Botrytis cinerea* and *Penicillium expansum,* and suggested that they could serve for protecting apples. No deposits of B66 and B90 strains are publicly available.

Sholberg P.L. et al. *Phytopathology,* 1994, Vol. 84, No, 10, page 1152, describe in a very short abstracts that several strains of *Pseudomonas sp., Enterobacter aero genes* and an unidentified yeast inhibited apple fruit decay by *Penicillum expansum.* No deposits are publicly available.

Gulati. M.K. et al *Mitt. Biol. Bundesanstalt Landforstwirtschaft,* 1998, No. 357, page 338, describe a test to control plant pathogens causing red root rot made with a concrete strain of *Erwinia herbicola* (G-584). Poor results are reported and no public deposit is available.

Dock L.L. et al. Journal of Food Protection, 1998, Vol. 61 (12), pages 1661-1665, describe that a non-identified strain of *Erwinia sp.,* isolated from onions in Danemark, can act as an agent to control *Botrytis cinerea* growth on apples stored under modified atmosphere. No public deposit is available.

El-Goorani et al. J. Phytopathology, 1992, Vol 136, pages 335-339, refers to nine strains of *E. herbicola* (*Pantoea agglomerans*) obtained from a personal culture collection not publicly available. Said *E. herbicola* strains are antibiotic producer and its activity is tested against *E. amilovora,* a plant pathogen not a fruit pathogen, which causes fire blight in rosaceous hosts.

US-A-5783411 refers to the use of a strain of *Pantoea agglomerans,* with deposit number NRL B21098, as an inhibitor to dry rot disease of potatoes. An *Erwinia herbicola* strain of ananus variety, called M232A by its inventors, is described in patent US-A-4045910, and placed in the American Collection of Standard Cultures with the identification number ATCC 31225, which is used for combatting the risk of freezing in plants.

An *Erwinia herbicola* strain, called EHO-10 by its inventors, is described in patent US-A-4569841, and placed in the American Collection of Standard Cultures with the identification number ATCC 39368, which is used for combatting bacterial diseases in plants and in particular, in the Bacterial Fire but it is not used for combatting fungic diseases in fruits.

### Object of the invention

The object of the present invention is to provide a substantially pure biological harvest of a new strain of the bacterium *Pantoea agglomerans (Erwinia herbicola)* capable of acting as a highly effective antagonist in biological control of the pathogenic fungi responsible for the rotting of pre and post-harvest fruit and which retain their effectiveness under conditions of low temperature and low-oxygen atmosphere.

A further object of the present invention consists in the utilization of said antagonist, alone or together with other antifungic agents and / or adjuvants to prevent the rotting of the harvested fruit during its storage.

Yet another object of the invention consists in providing a method for better preservation of the harvested fruit under various storage conditions in relation to temperature and atmosphere of oxygen and carbon dioxide.

### Brief description of the drawings

For a better understanding of same, this description is accompanied by 10 sheets of drawings, without limiting effects, for which a brief outline is provided. It must be taken into account that when dealing with the preservation under conventional cold conditions, it refers to temperature conditions equal to or lower than 5°C and a concentration of oxygen typical of the ambient atmosphere without any modification.
**Figure 1** is a graphic representation of the growth curve of the CECT-4920 strain, in NYDB medium and peptone medium, under preservation conditions of 1°C.
**Figure 2** is a graphic representation of the growth curve of the CECT-4920 strain, in NYDB medium and peptone medium, under preservation conditions of 30°C.
**Figure 3** is the graphic representation of the population development of the CECT-4920 strain, applied to injuries of 3 x 3 x 3 mm³ of "Golden Delicious" apples under preservation conditions of 20° and 30°C.
**Figure 4** is a three-dimensional bar-diagram view of the percentages of reduction of rotting in "Rocha" pears inoculated with *Botrytis cinerea* 10³, 10⁴ and 10⁵ c.f.u./ml, caused by inoculation of the CECT-4920 antagonist. Data obtained following 7 days of incubation at 20°C.
**Figure 5** is a three-dimensional bar-diagram view of the percentages of reduction of rotting in "Blanquilla" pears inoculated with *Penicillium expansum* 10³, 10⁴ and 10⁵ c.f.u./ml, caused by inoculation of the CECT-4920 antagonist. Data obtained following 7 days of incubation at 20°C.
**Figure 6** is a three-dimensional bar-diagram view of the percentages of reduction of rotting in "Conference" pears inoculated with *Rhizopus stolonifer* 10³, 10⁴ and 10⁵ c.f.u./ml, caused by inoculation of the CECT-4920 antagonist. Data obtained following 7 days of incubation at 20°C.
**Figure 7** is a three-dimensional bar-diagram view of the percentages of rotten fruits in "Valencia Late" oranges inoculated with *Penicillium digitatum* 10⁵ and 10⁶ c.f.u./ml, caused by inoculation of the CECT-4920 antagonist. Data obtained following 7 days of incubation at 20°C.
**Figure 8** is a three-dimensional bar-diagram view of the percentages of rotten fruits in "Valencia Late" oranges inoculated with *Penicillium italicum* 10⁵ and 10⁶ c.f.u./ml, caused by inoculation of the CECT-4920 antagonist. Data obtained following 7 days of incubation at 20°C.
**Figure 9** is a three-dimensional bar-diagram view of the percentages of reduction of rotting in "Early Red One" apples inoculated with *Penicillium expansum*, caused by inoculation of the CECT-4920 antagonist. Data obtained following 30 days of incubation under conventional cold conditions.
**Figure 10** is a three-dimensional bar-diagram view of the percentages of reduction of rotting in "Fuji" apples inoculated with *Penicillium expansum,* caused by inoculation of the CECT-4920 antagonist. Data obtained following 30 days of incubation under conventional cold conditions.
**Figure 11** is a three-dimensional bar-diagram view of the percentages of reduction of rotting in "Alejandrina" pears inoculated with *Penicillium expansum,* caused by inoculation of the CECT-4920 antagonist. Data obtained following 30 days of incubation under conventional cold conditions.
**Figure 12** is a three-dimensional bar-diagram view of the percentages of reduction of rotting in "Decana de Comice" pears inoculated with *Penicillium expansum,* caused by inoculation of the CECT-4920 antagonist. Data obtained following 30 days of incubation under conventional cold conditions.
**Figure 13** is a three-dimensional bar-diagram view of the percentages of reduction of rotting in "Golden Delicious" apples inoculated with *Botrytis cinerea,* caused by inoculation of the CECT-4920 antagonist. Data obtained following 30 days of incubation under conventional cold conditions.
**Figure 14** is a three-dimensional bar-diagram view of the percentages of reduction of rotting in "Golden Delicious" apples inoculated with *Penicillium expansum*, caused by bathing the fruits with the CECT-4920 antagonist. Data obtained following 90 days of incubation under controlled atmosphere: 1% O₂ - 1% CO₂; 2% O₂ - 1% CO₂; 2% O₂ - 2% CO₂; 2% O₂ - 4% CO₂; 2% O₂ - 6% CO₂; 3% O₂ - 3% CO₂; 3% O₂ - 6% CO₂.
**Figure 15** is a three-dimensional bar-diagram view of the percentages of reduction of rotting in "Valencia Late" oranges without artificial inoculation of pathogen, caused by inoculation of the CECT-4920 antagonist. Data obtained following 60 days of incubation at 3,5° C at different conditions under controlled atmosphere: 1.5% O₂ - 1.5% CO₂ ; 3% O₂ - 3% CO₂; 5% O₂ - 3% CO₂ and 8% O₂ - 5% CO₂;
**Figure 16** is a three-dimensional bar-diagram view of the percentages of reduction of rotting in "Blanquilla" pears, inoculated with *Penicillium expansum* caused by bathing the fruits with the CECT-4920 antagonist. Data obtained following 90 days of incubation under conventional cold conditions.
**Figure 17** is a three-dimensional bar-diagram view of the percentages of reduction of rotting in "Golden Delicious" apples, inoculated with *Botrytis cinerea* caused by bathing the fruits with the CECT-4920 antagonist. Data obtained following 90 days of incubation under controlled atmosphere: 1% O₂ - 1% CO₂.
**Figure 18** is a three-dimensional bar-diagram view of the percentages of reduction of rotting in "Rocha" pears, inoculated with *Penicillium expansum* caused by spraying with 8 x 10⁷ c.f.u./ml of the CECT-4920 antagonist and by application of the chemical fungicide Imazalil at a commercial dose. Comparative data obtained following 5.5 months of incubation at temperature of 1° C.
**Figure 19** is a three-dimensional bar-diagram view of the percentages of reduction of rotting in "Valencia Late" oranges, inoculated with *Penicillium digitatum* 10⁵ and 10⁶ c.f.u./ml, caused by inoculation of the CECT-4920 antagonist and by the food additive, sodium bicarbonate. Data obtained following 7 days of incubation at 20° C.

### Description of the invention

The authors of the present invention have managed to isolate a new strain of the species known, at present, as *Pantoea agglomerans* (Beijerinck, 1988) Gavini, Mergaert, Beji, Mielcarek, Izard, Kersters and De Ley, 1989, but also known by its synonyms *Erwinia herbicola* (Löhnis, 1911) Dye 1964, *Enterobacter agglomerans* (Beijerinck, 1988) Ewing and Fife, 1972 and *Erwinia milletiae* (Kawakami and Yoshida, 1920) Magrou, 1937 which shows very high effectiveness as an antagonist of the species of fungi which cause diseases in fruits before and after its harvesting (pre and post harvest), over a wide range of temperatures and oxygen atmospheres, which permits its industrial utilization for the biological control of said fungic species and the prevention of the rotting of the fruit.

The strain object of the present invention was isolated on the surface of apples by means of repeated washings with sterile water followed by a bathing in ultrasounds with a phosphate tampon, and a culture of same was deposited, in accordance with the provisions of the Treaty of Budapest on the recognition of deposition of microorganisms for the purpose of patent procedures, under the international depositing authority of Colección Española de Cultivos Tipo (Spanish Collection of Standard Cultures), situated in the Faculty of Biological Sciences, University of Valencia, 46100 Burjasot (Valencia), Spain, which assigned it deposit number CECT-4920.

The isolate of CECT-4920 was identified as *Pantoea agglomerans* and after its isolation it was cultivated in the peptone-agar solid medium, consisting in peptone, starch, dextrose and agar, in the peptic liquid medium consisting in peptone, starch and dextrose and in the NYDB liquid medium, consisting in culture of extract of yeast and dextrose.

Morphologically, the CECT-4920 strain forms yellow, round well-defined and mobile colonies. It has an aerobic growth in peptone-agar medium at 30° C, and an anaerobic growth in thioglicolate medium at 30°, and presents the biochemical characteristics detailed in Tables I and II:

**Table I**

| **-Results taken following 24 and 48 hours of the API 20 E- System of identification for Enterobacteriaceae and other negative-Gram bacillus at 30°C.** | | |
|---|---|---|
| ***Substrata (TESTS)*** | ***Reactions*/*enzymes*** | ***Results*** |
| Orthonitrophenol-galactoside (ONPG) | β-galactosidase | ++ |
| Arginine (ADH) | Dehydrolase arginine | - |
| Lysine (LDC) | *Decarboxylase* lysine | - |
| Ornithine (ODC) | *Decarboxylase* ornithine | - |
| Sodium citrate (CIT) | Citrate use | + |
| Sodium thiosulphate (H₂S) | H₂S production | - |
| Urea (URE) | Urease | - |
| Tryptophane (TDA) | Deaminase tryptophane | - |
| Tryptophane (IND) | Indole production | - |
| Sodium pyruvic (VP) | Acetoin production | + |
| Kohn gelatine (GEL) | Gelatinase | + |
| Glucose (GLU) | Fermentation / oxidation (4) | ++ |
| Manitol (MAN) | Fermentation / oxidation (4) | ++ |
| Inositol (INO) | Fermentation / oxidation (4) | - ^{w} |
| Sorbitol (SOR) | Fermentation / oxidation (4) | - |
| Ramnose (RHA) | Fermentation / oxidation (4) | ++ |
| Saccharose (SAC) | Fermentation / oxidation (4) | ++ |
| Melibiose (MEL) | Fermentation / oxidation (4) | ++ |
| Amygdaline (AMY) | Fermentation / oxidation (4) | ++ |
| Arabinose (ARA) | Fermentation / oxidation (4) | ++ |
| On filter paper (OX) | Cytochrome oxidase | - |
| GLU tube (NO₃) | NO₂ production | + |
| GLU tube (NO₂) | Reduction TO gas N₂ | - |
| API M or microscopy (MOB) | Movility | + |
| Mac Conkey medium(McC) | Growth | + |
| Glucose (OF/O) | Open: Oxidation | + |
| Glucose (OF/F) | Closed: fermentation | + |
| (+ positive - negative w weak) | | |

**Table II**

| **- Results taken following 24 hours of the BIOLOG GN MICROPLATE at 30° - Tests of utilization of 95 carbon sources for negative- Gram microorganisms.** | | | |
|---|---|---|---|
| ***Substrata (TESTS)*** | ***Results*** | ***Substrata*** | ***Results*** |
| Water | | P-hydroxyphenylacetic acid | |
| α-cyclodextrine | | Itaconic acid | |
| Dextrine | + | α-ketobutyric acid | |
| Glycogen | + | α-ketoglutaric acid | |
| Tween 40 | + | α-ketovaleric acid | |
| Tween 80 | + | D, L-lactic acid | + |
| N-acetil-galactosamine | / | Malonic acid | + |
| N-acetil-D-glucosamine | + | Propionic acid | |
| Adonitol | | Quinic acid | + |
| L-arabinose | + | D-saccharic acid | + |
| D-arabitol | + | Sebacic acid | |
| Cellobiose | / | Succinic acid | + |
| Meso-erythrite | | Bromosuccinic acid | + |
| D-fructose | + | Succinic acid | |
| L-fucose | | Glucuronamide | + |
| D-galactose | + | Alaninamide | |
| Gentibiose | + | D-alanine | + |
| α-D-glucose | + | L-alanine | + |
| Minositol | + | L-alanil-glycine | + |
| α-D-lactose | | L-asparagine | + |
| Lactulose | | L-aspartic acid | + |
| Maltose | + | L-glutamic-acid | + |
| D-manitol | + | L-aspartic glycil acid | + |
| D-manose | + | L-glutamic glycil acid | + |
| D-melibiose | + | L-histidine | + |
| β-methyl-D-glucoside | + | L-hydroxyproline | |
| D-psicose | + | L-leucine | |
| D-rafinose | | L-ornithine | + |
| L-ramnose | + | L-phenylalanine | |
| D-sorbitol | | L-proline | + |
| Sucrose | + | L-pyroglutamic acid | |
| D-trehalose | + | D-serine | / |
| Turanose | + | L-serine | + |
| Xylitol | | L-threonine | |
| Methyl pyruvate | + | D,L-carnitine | |
| Mono-methyl succinate | + | γ-aminobutyric acid | + |
| Acetic acid | + | Urocanic acid | + |
| Cis-aconitic acid | + | Inosine | + |
| Citric acid | | Uridine | |
| Formic acid | + | Timidine | + |
| D-galactonic acid lactone | | Phenylethylamine | |
| D-galacturonic acid | + | Putrescine | |
| D-gluconic acid | + | 2-aminoethanol | |
| D-glucosaminic acid | / | 2,3-butanediol | |
| D-glucuronic acid | + | Glycerol | + |
| α-hydroxybutyric acid | / | D,L-α-glycerol-phosphate | + |
| β-hydroxybutyric acid | | Glucose-1-phosphate | + |
| γ-hydroxybutyric acid | | Glucose-6-phosphate | + |
| **(+ positive / doubtfully positive w weak blank: negative)** | | | |

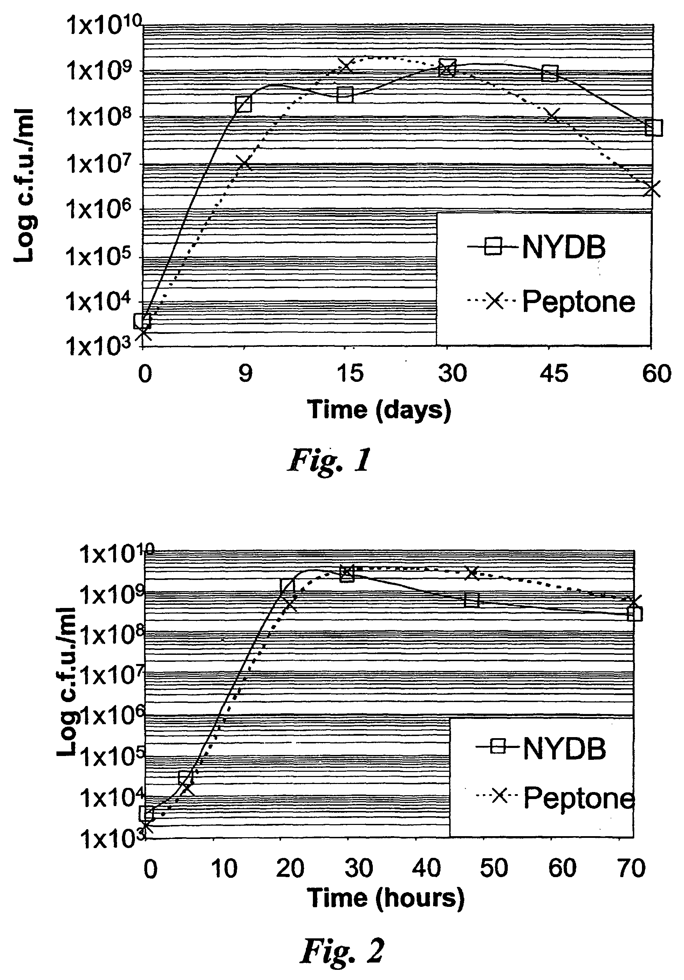

In vitro growth of the microorganism of the CECT-4920 strain, in NYDB medium and peptone medium, at initial pH 7, takes place under aerobic conditions at incubation temperatures between 1°C and 30°C. As it can be observed in Figures 1 and 2 the microorganism presents a similar growth in both mediums of culture, and the population maximum of 1°C is achieved after 15 days of incubation, and of 30°C after 30 hours of incubation, both in the peptone medium

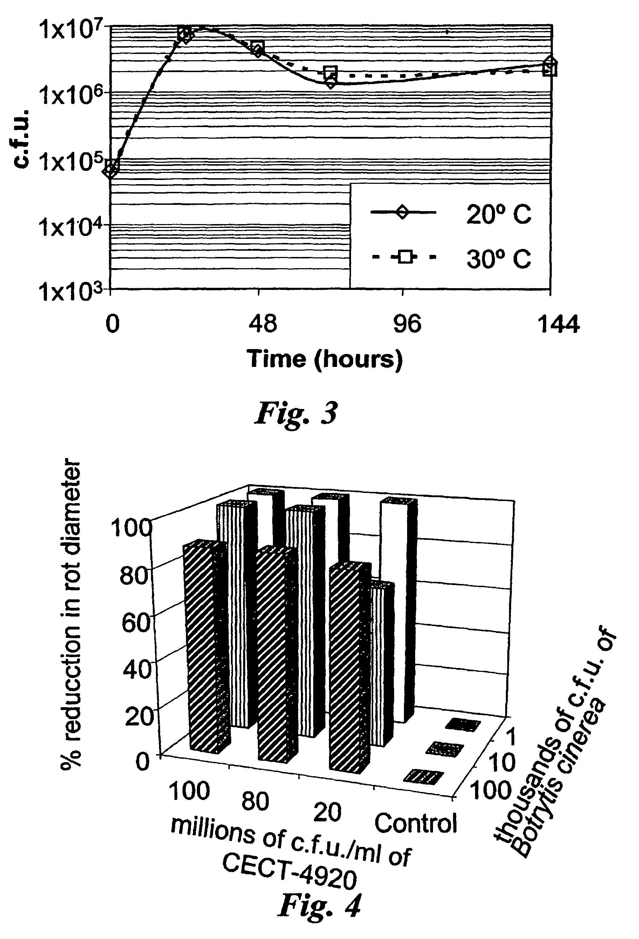

As shown by the graphic of Figure 3, the high growth of the CECT-4920 strain is shown in artificial injuries of the fruit where the antagonist must act, as the majority of the pathogenic fungi of the fruit need an injury to start the process of infection. A similar growth is observed at 20° C and at 30° C.

The inoculation of the microorganism on the surface of the fruit shows a high growth in conditions of controlled atmosphere with 3% oxygen and 3% carbon dioxide at temperature of 1° C, which are typical of the long storage conditions in fruit-horticultural centres.

The CECT-4920 isolate can be obtained, in laboratories and for industrial use, by cultivation thereof in a suitable medium, by means of conventional techniques sufficiently known by experts. It can be obtained, for example, by cultivation of the original strain in NYDB medium at pH 7, in a receptacle which can be shaken and aerated, at temperatures between 1° C and 37° C, for periods of time of the order of 20 to 50 hours. The population maximum (6 x 10⁹ c.f.u. /ml) for the NYDB medium is achieved at 24 hours at 30° C, in a six-litre-receptacle. Once the incubation period has finished the microorganisms are separated from the cultivation medium by conventional techniques of sedimentation, centrifuging or filtering, and the culture can be preserved by, for example, freezing in cryoballs.

The CECT-4920 antagonist can be applied to the surface of the fruits by any conventional technique. For example, a dispersion of the culture in water can be prepared and the fruit sprayed or sprinkled in the field before harvesting, or the treatment can be applied during the handling process of the harvested fruit, before storage of same, in which case the treatment can also be implemented by immersion.

The effective concentrations of the CECT-4920 antagonist in the dispersion of application for the treatment of fruits can vary in function of factors such as the type of fruit, its maturity, the concentration of the pathogenic fungus on the fruit, the storage temperature and humidity, etc. The range of effective concentrations is usually between 10⁷-2x10⁸ c.f.u./ml (colony-forming units per millilitre), what is considered a low concentration referring to bacteria, though said margins should not be taken to restrict the object of the present invention.

The isolate of CECT-4920 is very effective in the biological control of a large number of species of pathogenic fungi of fruits, including, though not restricted to *Botrytis cinerea, Penicillium digitatum, Penicillium expansum, Penicillium italicum* and *Rhizopus stolonifer*. Its effectiveness in the prevention of rotting of fruit is not limited to storage of same under ambient conditions of temperature and oxygen concentration, but, owing to the above-mentioned characteristics of the microorganism, its use also provides excellent results under the cold-storage and controlled atmosphere conditions habitually used by fruit-horticulture centres.

The effectiveness of the CECT-4920 isolate in the control of pathogenic fungi is comparable to that of the fungicide Imazalil, a chemical product derived from imidazol (1-[2-(2,4-dichlorophenyl)-2-(2-propenyloxi)ethyl]-1H-imidazol), one of the most widely used fungicides worldwide in the post-harvesting of fruits, which means that the isolate of CECT-4920 constitutes an effective alternative for said chemical product, with the advantage that it lacks the toxicity characteristics of Imazalil.

The CECT-4920 antagonist can be applied together with other antifungi agents and or adjuvants, waxes, food additives, such as sodium bicarbonate, and substances of low toxicity.

The CECT-4920 antagonist can only be used as a biological control agent for the pathogenic fungi in fruits, or can be used in combination with other synthetic antifungi agents or biological antagonists, for example with yeasts of the *Candida sake* species, such as the ones described in the Spanish patent ES-2089981 and in the North American patent application US-7-745796, aforementioned.

The isolate of CECT-4920 can be employed effectively to combat rotting of all types of fruits in any of their varieties, especially pip fruits, such as apples, pears and quinces; citrus fruits such as oranges, lemons and mandarins; stone fruits such as peaches, apricots, cherries and plums, in any of their varieties.

### Examples

The examples outlined below should be interpreted as an aid for a better understanding of the invention, and not as limitations to the object of it.

### Example 1. Making an aqueous dispersion of CECT-4920.

The CECT-4920 antagonist was implanted in a test tube with peptone-agar medium and incubated at 27°C for 24-48 hours. Then, from this test tube, it was implanted in an Erlenmeyer flask with 50 ml of NYDB medium which was incubated in an orbital agitator at 150 rpm and 30°C for 24 hours. The contents of the flask were then centrifuged at 7.500 rpm for 10 minutes and the floating part was removed. The sediment was dispersed in 50 ml of sterile distilled water and that dispersion was used to prepare the desired concentrations of the antagonist by calculating the transmittance of the microbial suspension in a spectrophotometer, as an indirect measurement of the concentration of the antagonist. The equivalence between the transmittance and the concentration of microorganisms was implemented through a recount of viables in Petri plate in peptone-agar medium. The concentrations are expressed, in each case, in c.f.u. /ml (colony-forming units per millilitre).

### Example 2. Effectiveness of CECT-10897 on pip fruits stored at ambient temperature.

The test was carried out on 3 varieties of healthy "Blanquilla", "Conference" and "Rocha" pears which were cleaned with water and left to dry, following which two perforations per fruit were made, of dimensions of approximately 3 x 3 x 3 mm³. The two incisions were situated on the same side of the pear, one on the top part and one on the bottom. The sample unit was made up of three pears, and three repetitions were made for each treatment.

The three fungic species tested were *Botrytis cinerea, Penicillium expansum, Rhizopus stolonifer,* and titration of the suspensions of spores of same was carried out using young 5-7 day cultures, implanted in PDA medium (potato, dextrose, agar) and incubated at temperature of 25°C, by scraping of the colonies into sterile distilled water with Tween 80. A spore count was then carried out in a Thoma chamber, setting the desired concentration, expressed in c.f.u./ml.

Batches of three pears prepared as explained above were inoculated with 25 µl of the suspension of CECT-4920 antagonist, at concentrations of 2 x 10⁷, 8 x 10⁷ and 1 x 10⁸ c.f.u./ml. Once the fruits were dry, they were inoculated with 20 µl of the titrated suspensions of pathogens of the three species selected, at concentrations of 10³, 10⁴ and 10⁵ c.f.u./ml. In parallel to this the control test was set up in which the suspension of the antagonist was substituted by sterile distilled water.

All the treated fruits were placed in alveoli, left to dry and then placed in boxes for incubation at 20°C with a relative humidity of 80%, for 7 days. The incubation period was set up on the basis of the time needed for the control pears to present large rotting diameters. Following said incubation period the results were read, by measuring the diameters of rot of all the perforations made.

The rot diameter readings at the various repetitions were subjected to a statistical analysis consisting in analysis of the variance and, once it was found that said variance analysis was significant (α<0.01 to α<0.05), a separation of averages was carried out using the Duncan Multiple Range Test, the results of which are expressed with the lower-case letters of the alphabet (a, b, c, d, etc.), in such a way that treatments with the same letter are statistically equal and treatments with different letters are statistically different.

Table III shows the results obtained in the case of biological control of *Botrytis cinerea* with "Rocha" pears, Table IV the results with *Penicillium expansum* with "Blanquilla" pears and Table V the results with *Rhizopus stolonifer* with "Conference" pears.

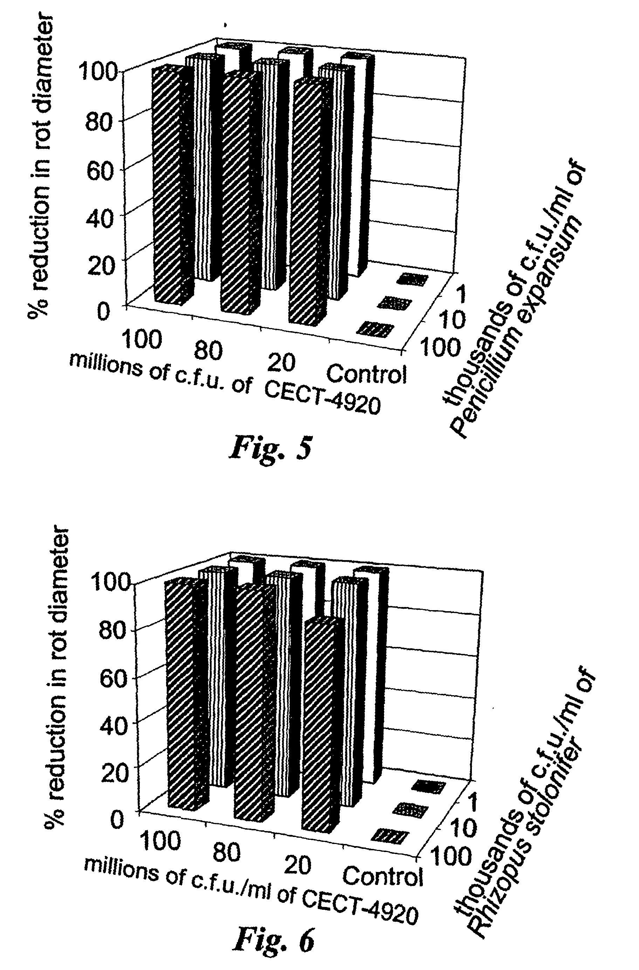

The graphic expression of the results of said tables are to be found in Figures 4, 5 and 6.

**Table III.**

| **Control of CECT-4920 on *B. cinerea* with "Rocha" pears.** | | | | | |
|---|---|---|---|---|---|
| **CONCENTRATION (c.f.u./ml) *B. cinerea*** | **MEASUREMENT** | **CECT-4920 DOSE c.f.u./ml** | | | |
| | | **Control 0** | **2 x 10**^{**7**} | **8 x 10**^{**7**} | **1 x 10**^{**8**} |
| **10**^{**3**} | **Average rot diameter (cm)** | 4.28 a | 0 b | 0 b | 0 b |
| | **% reduction in rot diameter** | - | 100 | 100 | 100 |
| **10**^{**4**} | **Average rot diameter (cm)** | 2.31 a | 0.71 b | 0 c | 0 c |
| | **% reduction in rot diameter** | - | 69 | 100 | 100 |
| **10**^{**5**} | **Average rot diameter (cm)** | 3.07 a | 0.49 b | 0.37 b | 0.38 b |
| | **% reduction in rot diameter** | - | 84 | 88 | 88 |

**Table IV.**

| **Control of CECT-4920 on *P. expansum* on "Blanquilla" pears.** | | | | | |
|---|---|---|---|---|---|
| **CONCENTRATION (c.f.u./ml) *P. expansum*** | **MEASUREMENT** | **CECT- 4920 DOSE c.f.u./ml** | | | |
| | | **Control 0** | **2 x 10**^{**7**} | **8 x 10**^{**7**} | **1 x 10**^{**8**} |
| **10**^{**3**} | **Average rot diameter (cm)** | 1.38 a | 0 b | 0 b | 0 b |
| | **% reduction in rot diameter** | - | 100 | 100 | 100 |
| **10**^{**4**} | **Average rot diameter (cm)** | 2.88 a | 0 b | 0 b | 0 b |
| | **% reduction in rot diameter** | - | 100 | 100 | 100 |
| **10**^{**5**} | **Average rot diameter (cm)** | 3.25 a | 0 b | 0 b | 0 b |
| | **% reduction in rot diameter** | - | 100 | 100 | 100 |

**Table V.**

| **Control of CECT-4920 on *R. stolonifer* with "Conference" pears.** | | | | | |
|---|---|---|---|---|---|
| **CONCENTRATION (c.f.u./ml) *R.stolonifer*** | **MEASUREMENT** | **CECT- 4920 DOSE c.f.u./ml** | | | |
| | | **Control 0** | **2 x 10**^{**7**} | **8 x 10**^{**7**} | **1 x 10**^{**8**} |
| **10**^{**3**} | **Average rot diameter (cm)** | 12 a | 0 b | 0 b | 0 b |
| | **% reduction in rot diameter** | - | 100 | 100 | 100 |
| **10**^{**4**} | **Average rot diameter (cm)** | 9.67 a | 0 b | 0 b | 0 b |
| | **% reduction in rot diameter** | - | 100 | 100 | 100 |
| **10**^{**5**} | **Average rot diameter (cm)** | 12 a | 1.33 b | 0 b | 0 b |
| | **% reduction in rot diameter** | - | 89 | 100 | 100 |

The data shown in the above-mentioned Tables III, IV and V, together with their graphic representation in Figures 4,5 and 6, show the high effectiveness of the CECT-4920 antagonist in three varieties of pears, in control of the three species of pathogenic fungi tested, and, consequently, in control of rotting of the fruits.

### Example 3.- Effectiveness of CECT-4920 on citrus fruits stored at ambient temperature.

The test was carried out with healthy "Valencia Late" oranges which were cleaned with water and left to dry, following a desinfection with a sodium hypochlorite solution at a concentration of chlorine of 0.5%, washed again and left to dry. Once the fruits were dry, a perforation per orange was made, of dimensions of approximately 5 mm length and 2 mm depth. The incision was situated in the equatorial part of the orange. The sample unit was made up of five oranges, and four repetitions were made for each treatment.

The two fungic species tested were *Penicillium digitatum* and *Penicillium italicum,* and titration of the suspensions of spores of same was carried out using the same way as for example 2, aforementioned.

Batches of five oranges prepared as explained above were inoculated with 25 ∝l of the suspension of pathogens of the two species selected, at concentrations of 10⁵ c.f.u./ml and 10⁶ c.f.u./ml. Once the fruits were dry, a spraying of the suspension of CECT-4920 antagonist took place for 5 seconds at concentrations of 4 x 10⁷ and 3 x 10⁸ c.f.u./ml. In parallel to this the control test was set up, in which the suspension of the antagonist was substituted by sterile distilled water.

All the treated fruits were placed in alveoli, left to dry and then placed in boxes for incubation at 20°C, with a relative humidity of 80%, for 7 days. Following said incubation period the results were read, by counting the number of rotten fruits.

The reading data of the number of rotten fruits of the various repetitions were subjected to a statistical analysis in the same way as for example 2 and set out in Tables VI and VII.

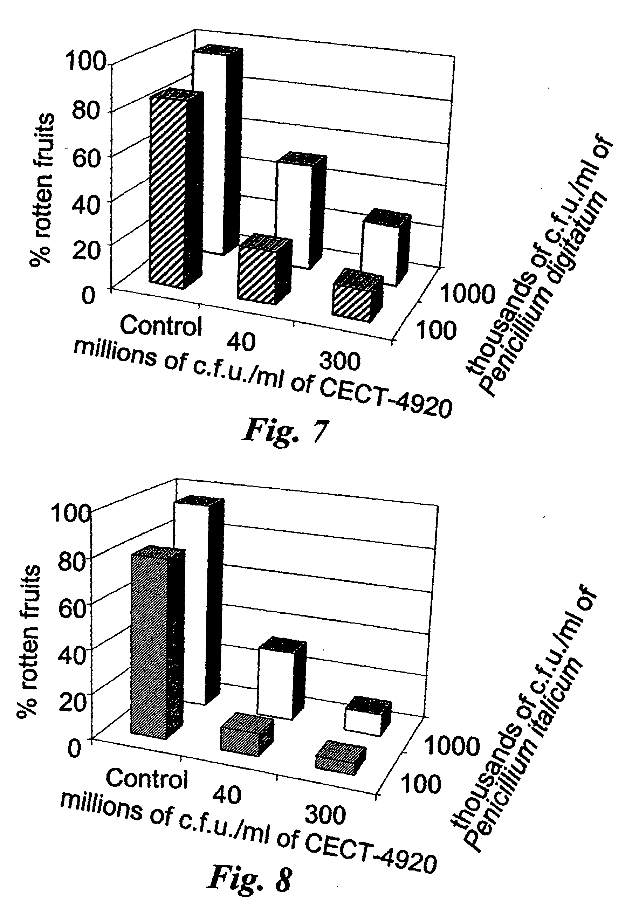

The graphic expression of the results of said tables are to be found in Figures 7 and 8, respectively.

**Table VI.**

| **Control of CECT-4920 on *P. digitatum* with "Valencia Late " oranges.** | | | | |
|---|---|---|---|---|
| **CONCENTRATION (c.f.u./ml.) *P. digitatum*** | **MEASUREMENT** | **CECT- 4920 DOSE c.f.u./ml** | | |
| | | **Control 0** | **4 x 10**^{**7**} | **3 x 10**^{**8**} |
| **10**^{**5**} | **% rotten fruits** | 85 a | 24 b | 14 c |
| **10**^{**6**} | **% rotten fruits** | 96 a | 50 b | 28 c |

**Table VII.**

| **Control of CECT-4920 on *P. italicum* with "Valencia Late" oranges.** | | | | |
|---|---|---|---|---|
| **CONCENTRATI ON (c.f.u./ml) *P. italicum*** | **MEASUREMENT** | **CECT- 4920 DOSE c.f.u./ml** | | |
| | | **Control 0** | **4 x 10**^{**7**} | **3 x 10**^{**8**} |
| **10**^{**5**} | **% rotten fruits** | 80 a | 10 b | 5 c |
| **10**^{**6**} | **% rotten fruits** | 95 a | 32 b | 10 c |

The data shown in the above-mentioned Tables VI and VII, together with their graphic representation in Figures 7 and 8, show the high effectiveness of the CECT-4920 antagonist in control of the main species of pathogenic fungi in citrus, *Penicillium digitatum and Penicillium italicum.*

### Example 4.- Control of Penicillium expansum on fruits stored at cold-storage temperature.

Following the same methods as for example 2, some tests were carried out with ten fruits per repetition and three repetitions per treatment. The test was carried out with "Early Red One" and "Fuji" apples, and "Blanquilla", "Rocha", "Decana de Comice" and "Alejandrina" pears.

25 ∝l of aqueous dispersion of the CECT-4920 antagonist were inoculated, at two concentrations 8 x 10⁷ and 1 x 10⁸ c.f.u./ml, and 20 ∝1 of *Penicillium expansum* inoculant, at a concentration of 10⁴ c.f.u./ml. All the treated fruits were placed in alveoli for their incubation at 1° C, with a relative humidity of 90-98%, for 30 days. Following said incubation period the results were read, by measuring the diameters of rot of all perforations made.

The results obtained are set out in Tables VIII, IX, X and XI.

**Table VIII.**

| **Control of CECT-4920 on *P. expansum* with "Early Red One" apples.** | | | | |
|---|---|---|---|---|
| **CONCENTRATION (c.f.u./ml) *P. expansum*** | **MEASUREMENT** | **CECT- 4920 DOSE c.f.u./ml** | | |
| | | **Control 0** | **8 x 10**^{**7**} | **1 x 10**^{**8**} |
| **10**^{**4**} | **Average rot diameter (cm)** | 1.11 a | 0.11 b | 0.19 b |
| | **% reduction in rot diameter** | - | 90 | 83 |

**Table IX.**

| **Control of CECT-4920 on *P. expansum* with "Fuji" apples.** | | | | |
|---|---|---|---|---|
| **CONCENTRATION (c.f.u./m 1) *P. expansum*** | **MEASUREMENT** | **CECT- 4920 DOSE c.f.u./ml** | | |
| | | **Control 0** | **8 x 10**^{**7**} | **1 x 10**^{**8**} |
| **10**^{**4**} | **Average rot diameter (cm)** | 0,46 a | 0,21 b | 0,04 c |
| | **% reduction in rot diameter** | - | 55 | 91 |

**Table X.**

| **Control of CECT-4920 on *P. expansum* with "Alejandrina" pears.** | | | | |
|---|---|---|---|---|
| **CONCENTRATION (c.f.u./ml) *P. expansum*** | **MEASUREMENT** | **CECT- 4920 DOSE c.f.u./ml** | | |
| | | **Control 0** | **8 x 10**^{**7**} | **1 x 10**^{**8**} |
| **10**^{**4**} | **Average rot diameter (cm)** | 1.12 a | 0.26 b | 0.20 b |
| | **% reduction in rot diameter** | - | 77 | 82 |

**Table XI.**

| **Control of CECT-4920 on P. expansum with "Decana de Comice" pears.** | | | | |
|---|---|---|---|---|
| **CONCENTRATION (c.f.u./ml) *P. expansum*** | **MEASUREMENT** | **CECT- 4920 DOSE c.f.u./ml** | | |
| | | **Control 0** | **8 x 10**^{**7**} | **1 x 10**^{**8**} |
| **10**^{**4**} | **Average rot diameter (cm)** | 1.16 a | 0.17 b | 0.35 b |
| | **% reduction in rot diameter** | - | 85 | 66 |

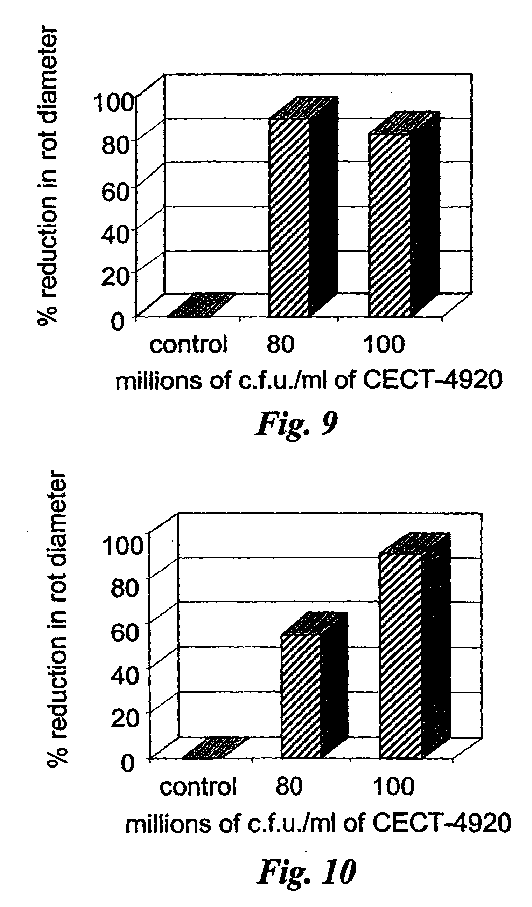

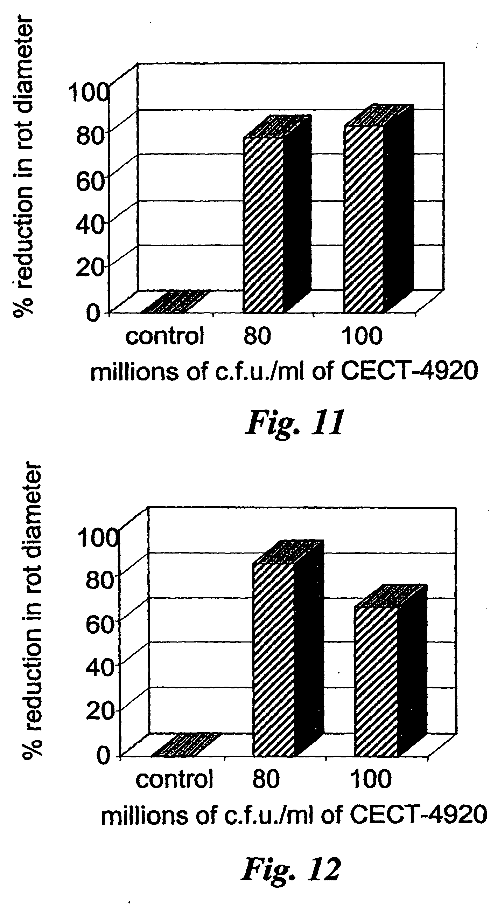

As it can be observed in the above tables and in Figures 9, 10, 11 and 12, the results obtained show a high degree of efficacy in the control of *Penicillium expansum* under cold-storage conditions, in the different varieties of pears and apples, some of them recently introduced into the European fruit market.

### Example 5.- Control of Botrytis cinerea on fruits stored at cold-storage temperature.

A test was carried out, under the same conditions as example 4, using 20 ∝l of *Botrytis cinerea* inoculant as inoculant of the pathogenic fungus at a concentration of 10⁴ c.f.u./ml.

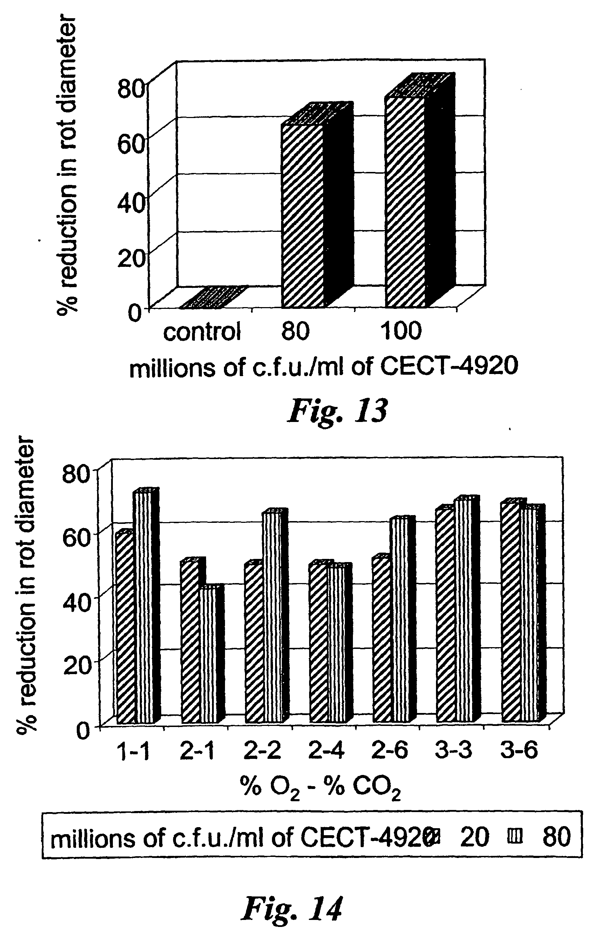

The results obtained, following the above-mentioned statistical processing, are set out in Table XII, with graphic representation thereof in Figure 13.

**Table XII.**

| **Control of CECT-4920 on *B. cinerea* with "Golden Delicious" apples.** | | | | |
|---|---|---|---|---|
| **CONCENTRATION (c.f.u./ml) *B. cinerea*** | **MEASUREMENT** | **CECT-4920 DOSE c.f.u./ml** | | |
| | | **Control 0** | **2 x 10**^{**7**} | **8 x 10**^{**7**} |
| **10**^{**4**} | **Average rot diameter (cm)** | 9.40 a | 3.27 b | 2.38 c |
| | **% reduction in rot diameter** | - | 65 | 75 |

The rot diameter reduction results were satisfactory, especially at a concentration of the CECT-4920 antagonist of 8 x 10⁷ c.f.u./ml.

### Example 6.- Effectiveness of CECT-4920 against Penicillium expansum on fruits stored at low temperatures and different oxygen and carbon dioxide atmosphere.

For this test the dispersion of antagonist was prepared on a larger scale, in the following manner:

The CECT-4920 antagonist was implanted in a test tube with peptone-agar medium and was incubated at 27°C for 24-48 hours. The contents of this test tube were then implanted in a fermenter fitted with an agitator and an aeration system, with 6,000 ml of NYDB medium and incubated at 30°C for 24 hours. The contents of the fermenter were then centrifuged at 7.500 rpm for 10 minutes and the floating matter was removed. The sediment was dispersed in sterile distilled water and that dispersion was used to prepare the desired concentrations in the same way as for example 1.

Fifteen "Golden Delicious" apples were used per repetition and three repetitions per treatment. Two perforations were made in each apple with a scalpel. The two incisions were situated in the equatorial part of the fruit, in its opposite faces.

The apples were treated with the CECT-4920 antagonist, by immersing the fruits into baths containing dispersions of antagonist at concentrations of 2 x 10⁷ and 8 x 10⁷ c.f.u./ml. An untreated control batch of forty-five apples was used. Then, once the apples were dry, they were lowered into a bath with a titrated dispersion of *Penicillium expansum* at a concentration of 10⁴ c.f.u./ml.

All the fruits were placed in alveoli, and divided into five different batches, stored for 90 days at 1° C in cold microcameras with the following gas conditions: 1% O₂ - 1% CO₂; 2% O₂ - 1% CO₂; 2% O₂ - 2% CO₂; 2% O₂ - 4% CO₂; 2% O₂ - 6% CO₂; 3% O₂ - 3% CO₂; 3% O₂ - 6% CO₂.

The results were read and processed statistically as it was above-mentioned, and are set out in Table XIII, with graphic representation thereof in Figure 14.

**Table XIII.**

| **Control of CECT-4920 on *P. expansum* with "Golden Delicious" apples under different conditions of controlled atmosphere.** | | | | |
|---|---|---|---|---|
| **GAS CONCENTRATION %O**_{**2**} **- %CO**_{**2**} | **MEASUREMENT** | **CECT-4920 DOSE c.f.u./ml** | | |
| | | **Control 0** | **2 x 10**^{**7**} | **8 x 10**^{**7**} |
| **1%-1%** | **Average rot diameter (cm)** | 3,72 a | 1,51 bc | 1,04 c |
| **2%-1%** | **Average rot diameter (cm)** | 2,92 a | 1,45 b | 1,72 ab |
| **2%-2%** | **Average rot diameter (cm)** | 2,54 a | 1,28 b | 0,90 b |
| **2%-4%** | **Average rot diameter (cm)** | 3.02 a | 1.58 b | 1.55 b |
| **2%-6%** | **Average rot diameter (cm)** | 2.89 a | 1.40 b | 1,07 b |
| **3%-3%** | **Average rot diameter (cm)** | 2.84 a | 0.96 b | 0.87 b |
| **3%-6%** | **Average rot diameter (cm)** | 2.48 a | 0.79 b | 0.85 b |

The analysis of the results shows a high effectiveness of the CECT-4920 antagonist in the control of *Penicillium expansum* in the most varied conditions of controlled atmosphere.

### Example 7.- Control of rotting on citrus stored at low temperature and different oxygen and carbon dioxide atmosphere.

The test was carried out with "Valencia Late" oranges, picked up from the field, that without any previous cleaning were perforated as it was explained in example 3. Forty fruits formed the sample unit and three repetitions were made per treatment.

No fungus was inoculated in this test; it refers to a natural inoculation as in the citrus fruits most of the pathogenic fungi come from the field.

The CECT-4920 antagonist was prepared on a large scale, following the model explained in example 6. The fruits were treated with the antagonist for 1 minute by lowering the fruits in a bath containing the dispersion of the CECT-4920 microorganism at a concentration of 1 x 10⁸ c.f.u./ml. A control test was prepared, in which the antagonist bath was substituted by a water bath.

All the fruits were placed in alveoli, and divided into five different batches, which were stored for 60 days at 3,5° C in cold microcameras with the following gas conditions : 1 . 5% O₂ - 1.5% CO₂ ; 3% O₂ - 3% CO₂ ; 5% O₂ - 3% CO₂ ; and 8% O₂ - 5% CO₂.

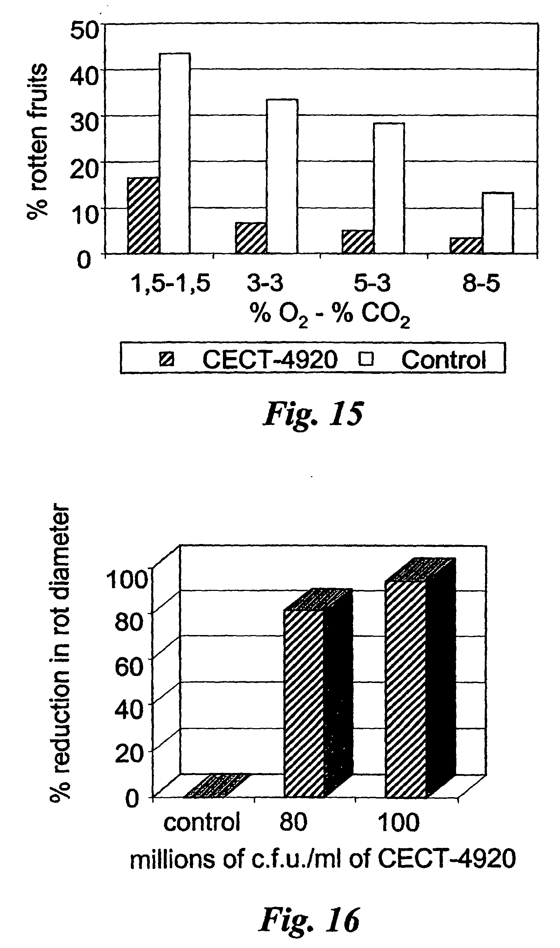

The results were read and processed statistically as it has been explained and shown in Table XIV. Their graphic representation is set out in figure 15.

**Table XIV.**

| **Effectiveness of CECT-4920 in the rotting control of citrus at different conditions of controlled atmosphere. (Test without artificial inoculation of pathogens).** | | | |
|---|---|---|---|
| **GAS CONCENTRATION %O**_{**2**} **- %CO**_{**2**} | **MEASUREMENT** | **CECT-4920 DOSE c.f.u./ml** | |
| | | **Control 0** | **1 x 10**^{**8**} |
| **1.5%-1.5%** | **% rotten fruits** | 43 a | 17 b |
| **3%-3%** | **rotten fruits** | 33 a | 7 b |
| **5%-3%** | **% rotten fruits** | 28 a | 5 b |
| **8%-5%** | **rotten fruits** | 13 a | 3 b |

From the analysis of the results, it can be concluded that the CECT-4920 antagonist presents a high effectiveness in the control of the pathogenic fungi in citrus at different controlled atmosphere conditions, achieving thus one of the most important objectives in the citrus sector, which tries to lengthen the conservation period, a very limited objective in conservation under conventional cold conditions.

### Example 8.- Control of pathogenic fungi in medium-scale tests.

For this test the dispersion of antagonist was prepared on a larger scale, following the method previously explained.

The test was carried out with "Blanquilla" pears and "Golden Delicious" apples, treated as explained in example 6. The sample unit was formed by twenty fruits and four repetitions were made per treatment.

Once the fruits were treated in the baths containing the dispersion of the CECT-4920 antagonist at concentrations of 2 x 10⁷ and 8 x 10⁷ c.f.u./ml and inoculated with the pathogenic fungus, through a bath with the titration of the suspensions of spores, they were separated into two batches and stored for 90 days: one batch under conventional cold (1°C and ambient oxygen) and another one under controlled atmosphere conditions (pear: 0.5° C and 3% oxygen; apple: 1°C and 1% oxygen).

The two tested fungic species were *Botrytis cinerea* and *Penicillium expansum* at the concentration of 10⁴ c.f.u./ml.

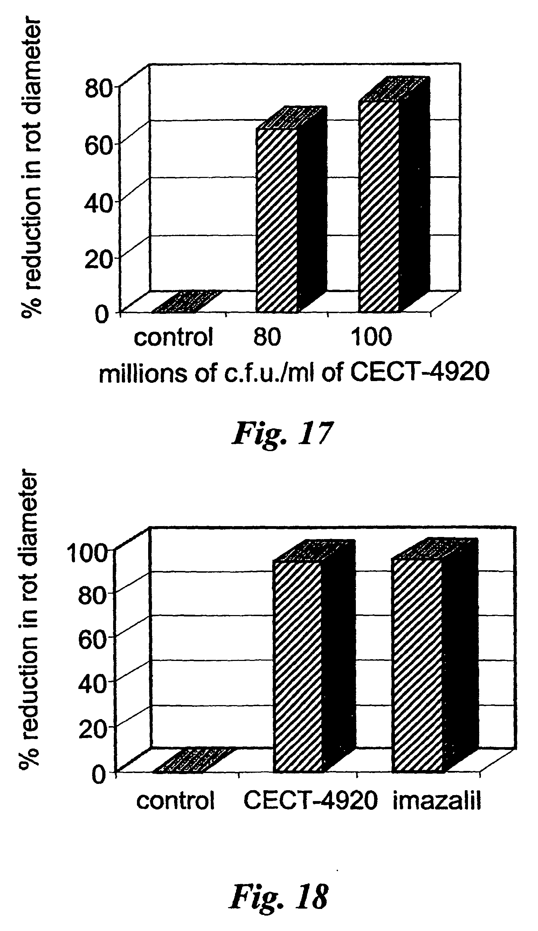

The results were read and processed in the same way as in the previous examples. As examples, the results of the *Penicillium expansum* control in "Blanquilla" pears, stored under controlled atmosphere, are shown in Table XV, and the *Botrytis cinerea* control in "Golden Delicious" apples, stored under conventional cold, are shown in Table XVI. Their graphic representations are set out in Figures 16 and 17.

**Table XV.**

| **Control of CECT-4920 on *P. expansum* with "Blanquilla" pears under controlled atmosphere.** | | | | |
|---|---|---|---|---|
| **CONCENTRATION (c.f.u./ml) *P. expansum*** | **MEASUREMENT** | **CECT-4920 DOSE c.f.u./ml** | | |
| | | **Control 0** | **2 x 10**^{**7**} | **8 x 10**^{**7**} |
| **10**^{**4**} | **Average rot diameter (cm)** | 0.39 a | 0.076 b | 0.026 b |
| | **% reduction in rot diameter** | - | 81 | 94 |

**Table XVI.**

| **Control of CECT-4920 on *B. cinerea* with "Golden Delicious" apples preserved under normal cold conditions.** | | | | |
|---|---|---|---|---|
| **CONCENTRATION (c.f.u./ml) *B. cinerea*** | **MEASUREMENT** | **CECT-4920 DOSE c.f.u./ml** | | |
| | | **Control 0** | **2 x 10**^{**7**} | **8 x 10**^{**7**} |
| **10**^{**4**} | **Average rot diameter (cm)** | 2.5 a | 0.84 b | 0.95 b |
| | **% reduction in rot diameter** | - | 66 | 67 |

As it can be observed in the above tables and in Figures 16 and 17, the results obtained show a high degree of efficacy in the control of pathogenic fungi, *Penicillium expansum* and *Botrytis* cinerea under low-oxygen atmosphere conditions, and conventional atmosphere, during a long period of time.

### Example 9.- Comparison with the fungicide Imazalil.

The test was carried out with "Rocha" pears following the method explained in example 6. Ten fruits formed the sample unit, and four repetitions were made per treatment.

Both CECT-4920 antagonist and Imazalil fungicide were applied by bathing the fruits in solutions or dispersions of the same. The antagonist was applied at a concentration of 8 x 10⁷ c.f.u./ml and the Imazalil at the commercial recommended dose. The pears were then inoculated with the pathogen of the species *Penicillium expansum,* by bathing them at a concentration of 10⁴ c.f.u./ml.

The fruits were incubated under conventional cold conditions for 5.5 months, at the end of which the results were read and processed statistically.

The results obtained are shown in Table XVII and their graphic representation is set out in Figure 18.

**Table XVII.**

| **Control of *P. expansum*, comparing with Imazalil.** | | | | |
|---|---|---|---|---|
| **CONCENTRATION (c.f.u./ml) *P. expansum*** | **MEASUREMENT** | **TREATMENT** | | |
| | | **CONTROL** | **CECT-4920** | **IMAZALIL** |
| **10**^{**4**} | **Average rot diameter (cm)** | 11.1 a | 0.7 b | 0.51 b |
| | **% reduction in rot diameter** | - | 94 | 95 |

It can be observed that the effectiveness of the CECT-4920 antagonist and the Imazalil is statistically the same after 5.5 months under cold-storage conservation.

### Example 10.- Effectiveness of the combined treatment of the CECT-4920 antagonist with a food additive in the rotting control with citrus stored at ambient temperature.

The fruits were prepared following the method explained in example 3, and the tests were carried out with twenty "Valencia Late" oranges per repetition and three repetitions per treatment.

The sodium bicarbonate was the selected food additive (NaHCO₃) at a concentration of 2%.

The oranges were inoculated with 25 ∝l of the titrated suspension of *Penicillium digitatum* at a concentration of 10⁶ c.f.u./ml. The inoculated fruits were then immersed in the sodium bicarbonate solution for 2.5 minutes. Once the fruits were dry, they were then immersed in the solution of the CECT-4920 antagonist, at a concentration of 1 x 10⁸ c.f.u./ml, for one minute.

In parallel, after the inoculation of the pathogen, the three control tests were set up, consisting in the immersion of the fruit in the following solutions: sodium bicarbonate at 2% for 2.5 minutes; CECT-4920 antagonist at the concentration of 1 x 10⁸ c.f.u./ml, for one minute; running water for one minute.

After the fruits were dry, they were placed in alveoli for their incubation at 20°C, with a relative humidity of 80%, for seven days. Following said incubation period the results were read in the same way as in the previous examples.

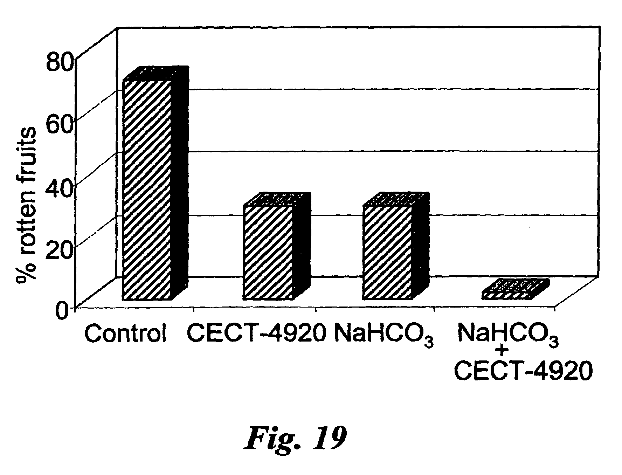

The results obtained are set out in Table XVIII and their graphic representation in Figure 19.

**Table XVIII.**

| **Control of CECT-4920 combined with sodium bicarbonate on *P. digitatum* with "Valencia Late" oranges.** | | | | | |
|---|---|---|---|---|---|
| **CONCENTRATION (c.f.u./ml) *P. digitatum*** | **MEASUREMENT** | **TREATMENTS TREATMENTS** | | | |
| | | **Control (Water)** | **CECT-4902** | **Sodium bicarbonate** | **CECT-4920 + Sodium bicarbonate** |
| **10**^{**6**} | **% rotten fruits** | 70 a | 30 b | 30 b | 2 c |

The results obtained show a high degree of efficacy of the CECT-4920 antagonist, which is significantly improved when the antagonist treatment is made together with the food additive.

### Information on dipositing of the CECT-4920 strain.

The microorganism was deposited in accordance with the provisions of the Treaty of Budapest on recognition of the depositing of microorganisms for the purpose of patent procedure, at the international depositing authority of Colección Española de Cultivos Tipo (CECT), situated at the Department of Microbiology, Faculty of Biological Sciences, University of Valencia, 46100 Burjasot (Valencia) Spain. The deposit was made on the 11th July 1997 and the CECT assigned it deposit number CECT-4920.

The deposit is at the disposal of the public, under the conditions provided for in the aforesaid Treaty of Budapest, although this availability cannot be interpreted as a licence to put into practice the object of the present invention, infringing the rights of the applicant for the present patent.

## Claims

1. The substantially pure biological culture of the strain of the species *Pantoea agglomerans,* deposited with number CECT-4920.

2. Use of the culture CECT-4920 of Claim 1 as an antagonist for the biological control of the pathogenic fungi responsible for the rotting of fruits.

3. Use, according to Claim 2, **characterized in that** the pathogenic fungi belong to any of the species *Botrytis cinerea, Penicillium digitatum, Penicillium expansum, Penicillium italicum and Rhizopus stolonifer.*

4. Use, according to Claim 2, **characterized in that** fruits are those known as pome fruits, especially apples, pears and quinces, in any of their varieties.

5. Use, according to Claim 2, **characterized in that** the fruits are citrus, especially oranges, lemons and mandarins, in any of their varieties.

6. Use, according to Claim 2, **characterized in that** the fruits are those known as stone fruits, especially peaches, apricots, cherries and plums, in any of their varieties.

7. A method for preventing rotting of fruits, **characterized in that** the fruit is treated, before or after harvesting, with a preparation of the CECT-4920 culture of Claim 1.

8. A method, according to Claim 7, **characterized in that** the treatment is carried out by means of spraying, wetting or immersion of the fruits in or with an aqueous dispersion of the CECT-4920 antagonist.

9. A method, according to Claim 7, **characterized in that** the treatment is carried out with food additives together with an aqueous dispersion of the CECT-4920 antagonist.

10. A method, according to Claim 7, **characterized in that** the treatment is carried out with a mixture of an aqueous dispersion of the CECT-4920 antagonist together with one or more antagonist microorganisms of the pathogen fungi responsible for the rotting of fruits.

11. A method, according to Claim 8, **characterized in that** the concentration of the CECT-4920 antagonist in the aqueous dispersion ranges between 10⁷ and 2 x 10⁸ c.f.u./ml.

12. A method, as claimed according to Claim 7, **characterized in that** the storage conditions of the fruits after their harvesting are those of ambient temperature and oxygen.

13. A method, according to Claim 7, **characterized in that** the storage conditions of the fruits after their harvesting are those of temperature lower than 5°C and atmosphere with oxygen content lower than 8%.

## Patentansprüche

1. Die überwiegend reine biologische Kultur des Stamms der Spezies *Pantoea agglomerans,* hinterlegt unter der Nummer CECT-4920.

2. Verwendung der Kultur CECT-4920 gemäß Anspruch 1 als Antagonist zur biologischen Kontrolle von pathogenen Pilzen, die Verursacher der Fäulnis bei Obst sind.

3. Verwendung gemäß Anspruch 2, **dadurch gekennzeichnet, dass** die pathogenen Pilze zu einer der Spezies *Botrytis cinerea, Penicillium digitatum, Penicillium expansum, Penicillium italicum und Rhizopus stolonifer* gehören.

4. Verwendung gemäß Anspruch 2, **dadurch gekennzeichnet, dass** das Obst zum sogenannten Kernobst gehört, besonders Äpfel, Birnen und Quitten in allen ihren Varietäten.

5. Verwendung gemäß Anspruch 2, **dadurch gekennzeichnet, dass** das Obst zu den Zitrusfrüchten gehört, besonders Orangen, Zitronen und Mandarinen in allen ihren Varietäten.

6. Verwendung gemäß Anspruch 2, **dadurch gekennzeichnet, dass** das Obst zum sogenannten Steinobst gehört, besonders Pfirsiche, Aprikosen, Kirschen und Pflaumen in allen ihren Varietäten.

7. Ein Verfahren zum Verhindern der Fäulnis bei Obst, **dadurch gekennzeichnet, dass** das Obst vor oder nach der Ernte mit einer Zubereitung aus der Kultur CECT-4920 gemäß Anspruch 1 behandelt wird.

8. Ein Verfahren gemäß Anspruch 7, **dadurch gekennzeichnet, dass** die Behandlung durch Bestäuben, Benetzen oder Eintauchen des Obstes in oder mit einer wässrigen Dispersion des Antagonisten CECT-4920 erfolgt.

9. Ein Verfahren gemäß Anspruch 7, **dadurch gekennzeichnet, dass** die Behandlung mit Lebensmittelzusätzen zusammen mit einer wässrigen Dispersion des Antagonisten CECT-4920 erfolgt.

10. Ein Verfahren gemäß Anspruch 7, **dadurch gekennzeichnet, dass** die Behandlung mit einer Mischung einer wässrigen Dispersion des Antagonisten CECT-4920 zusammen mit einem oder mehreren Mirkoorganismen, Antagonisten der pathogenen Pilze, erfolgt, welche die Fäulnis bei Obst verursachen.

11. Ein Verfahren gemäß Anspruch 8, **dadurch gekennzeichnet, dass** die Konzentration des Antagonisten CECT-4920 in der wässrigen Dispersion zwischen 10⁷ und 2 x 10⁸ pfu/ml liegt.

12. Ein Verfahren gemäß Anspruch 7, **dadurch gekennzeichnet, dass** die Lagerung des Obstes nach der Ernte bei Umgebungstemperatur und in sauerstoffhaltiger Atmosphäre erfolgt.

13. Ein Verfahren gemäß Anspruch 7, **dadurch gekennzeichnet, dass** die Lagerung des Obstes nach der Ernte bei einer Temperatur unter 5°C und in einer Atmosphäre mit einem Sauerstoffgehalt von weniger als 8% erfolgt.

## Revendications

1. Culture biologique substantiellement pure de la souche de l'espèce *Pantoea agglomerans* déposée sous le numéro CECT-4920.

2. Utilisation de la culture CECT-4920 de la revendication 1 comme antagoniste pour le contrôle biologique des champignons pathogènes responsables du pourrissement des fruits.

3. Utilisation selon la revendication 2, **caractérisée en ce que** les champignons pathogènes appartiennent à l'une quelconque des espèces *Botrytis cinerea, Penicillium digitatum, Penicillium expansum, Penicillium italicum* et *Rhizopus stolonifer.*

4. Utilisation selon la revendication 2, **caractérisée en ce que** les fruits sont ceux connus comme fruits à pépins, en particulier les pommes, les poires et les coings, quelles que soient leurs variétés.

5. Utilisation selon la revendication 2, **caractérisée en ce que** les fruits sont des agrumes, en particulier des oranges, des citrons et des mandarines, quelles que soient leurs variétés.

6. Utilisation selon la revendication 2, **caractérisée en ce que** les fruits sont ceux connus comme fruits à noyau, en particulier les pêches, les abricots, les cerises et les prunes, quelles que soient leurs variétés.

7. Procédé pour empêcher le pourrissement des fruits **caractérisé en ce que** le fruit est traité, avant ou après récolte, avec une préparation de la culture CECT-4920 de la revendication 1.

8. Procédé selon la revendication 7, **caractérisé en ce que** le traitement est effectué au moyen d'une pulvérisation, d'une humidification ou d'une immersion des fruits dans ou avec une dispersion aqueuse de l'antagoniste CECT-4920.

9. Procédé selon la revendication 7, **caractérisé en ce que** le traitement est effectué avec des additifs alimentaires conjointement avec une dispersion aqueuse de l'antagoniste CECT-4920.

10. Procédé selon la revendication 7, **caractérisé en ce que** le traitement est effectué avec un mélange d'une dispersion aqueuse de l'antagoniste CECT-4920 conjointement avec un ou plusieurs microorganismes antagonistes des champignons pathogènes responsables du pourrissement des fruits.

11. Procédé selon la revendication 8, **caractérisé en ce que** la concentration en antagoniste CECT-4920 dans la dispersion aqueuse est comprise entre 10⁷ et 2 x 10⁸ c.f.u. /ml.

12. Procédé selon la revendication 7, **caractérisé en ce que** les conditions de conservation des fruits après leur récolte sont celles de la température et de l'oxygène ambiants.

13. Procédé selon la revendication 7, **caractérisé en ce que** les conditions de conservation des fruits après leur récolte sont celles d'une température inférieure à 5°C et d'une atmosphère avec une teneur en oxygène inférieur à 8%.
